# EUROPEAN PATENT SPECIFICATION

(11) **EP 3 658 523 B1**
(45) Date of publication and mention of the grant of the patent: **26.04.2023**
(21) Application number: 18759002.1
(22) Date of filing: 27.07.2018
(51) Int. Cl.: C07C 17/087, C07C 21/18

(54) **PROCESS FOR PREPARING (Z)-1,1,1,4,4,4-HEXAFLUORO-2-BUTENE**
VERFAHREN ZUR HERSTELLUNG VON (Z)-1,1,1,4,4,4-HEXAFLUOR-2-BUTEN
PROCÉDÉ DE PRÉPARATION DE (Z)-1,1,1,4,4,4-HEXAFLUORO-2-BUTÈNE

(30) Priority: 27.07.2017 US 201762537784 P
(43) Date of publication of application: 03.06.2020
(73) Proprietor: The Chemours Company FC, LLC, Wilmington, DE 19899 (US)
(72) Inventor: PENG, Sheng, Hockessin Delaware 19707 (US)
(74) Representative: Dannenberger, Oliver Andre
(86) International application number: PCT/US2018/044085
(87) International publication number: WO 2019/023572

(56) References cited:
- WO-A1-2014/052695
- WO-A1-2015/120250
- US-A1- 2016 039 728
- US-B2- 6 329 560

## Description

### TECHNICAL FIELD

This disclosure relates to processes and intermediates for preparing (Z)-1,1,1,4,4,4-hexafluoro-2-butene and compositions which may be useful in applications including refrigerants, high-temperature heat pumps, organic Rankine cycles, as fire extinguishing/fire suppression agents, propellants, foam blowing agents, solvents, and/or cleaning fluids.

### BACKGROUND

Many industries have been working for the past few decades to find replacements for the ozone depleting chlorofluorocarbons (CFCs) and hydrochlorofluorocarbons (HCFCs). The CFCs and HCFCs have been employed in a wide range of applications, including their use as aerosol propellants, refrigerants, cleaning agents, expansion agents for thermoplastic and thermoset foams, heat transfer media, gaseous dielectrics, fire extinguishing and suppression agents, power cycle working fluids, polymerization media, particulate removal fluids, carrier fluids, buffing abrasive agents, and displacement drying agents. In the search for replacements for these versatile compounds, many industries have turned to the use of hydrofluorocarbons (HFCs).

US 2016/0039728A1 discloses a process for the production of Z-1,1,1,4,4,4-hexafluoro-2-butene from 2,3-dichloro-1,3-butadiene comprising the step of chlorinating hydrogen fluoride and combining it with 1,1,2,3,4,4-hexachlorobuta-1,3-diene to provide E- or Z-1,1,1,4,4,4-hexafluoro-2-chloro-2-butene. A general procedure for this reaction step is described in US 6,329,560.

### SUMMARY

The present application provides, processes of preparing 2-chloro-1,1,1,4,4,4-hexafluoro-2-butene, comprising reacting hexachlorobutadiene with hydrofluoric acid in the presence of a transition metal catalyst, wherein the transition metal catalyst is a niobium catalyst selected from niobium (V) chloride, niobium (IV) chloride or a mixture thereof.

The present application further provides processes further comprising reacting 2-chloro-1,1,1,4,4,4-hexafluoro-2-butene with a base in the presence of an alkali metal halide and a quaternary (C₄₋₁₂ alkyl)ammonium salt to form perfluorobut-2-yne.

The present application further provides processes further comprising reacting perfluorobut-2-yne with hydrogen in the presence of a hydrogenation catalyst to form (Z)-1,1,1,4,4,4-hexafluoro-2-butene.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting.

### DETAILED DESCRIPTION

Antimony (V) catalysts have been used in anhydrous the HF fluorination of halo(hydro)carbons since the 1890s. However, there are notable issues associated with antimony catalysts including water toxicity. For example, water regulations for antimony are limited to approximately 6 ppb. Meeting these regulations requires significant consideration in the work-up of reaction mixtures involving antimony catalysts and subsequent waste handling. In addition, reduction of Sb(V) to Sb(III) is a highly favorable process which leads to rapid catalyst deactivation. For example, the use of SbCls requires a chlorine co-feed into the reaction to provide catalyst regeneration. Furthermore, antimony (V) pentafluoride catalyst is highly volatile, and high temperature operations are not suitable as the catalyst easily vaporizes and can plug reactor lines. Accordingly, the present application provides alternative transition metal catalysts useful in the HF fluorination of halo(hydro)carbons, particularly fluorination of hexachlorobutadiene (HCBD) to stereoselectively produce (Z)-2-chloro-1,1,1,4,4,4-hexafluoro-2-butene (*i.e.,* (Z)-1326mxz), a key intermediate in the preparation of (Z)-1,1,1,4,4,4-hexafluoro-2-butene which is useful in various applications (*e.g*., as a foam expansion agent or refrigerant) due to its low GWP, non-flammability, high efficiency, and thermal stability.

### Definitions

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, use of "a" or "an" are employed to describe elements and components described herein. This is done merely for convenience and to give a general sense of the scope of the invention. This description should be read to include one or at least one and the singular also includes the plural unless it is obvious that it is meant otherwise.

As used herein, the term "about" is meant to account for variations due to experimental error (*e.g.*, plus or minus approximately 10% of the indicated value).

When an amount, concentration, or other value or parameter is given as either a range, preferred range or a list of upper preferable values and/or lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range.

Global warming potential (GWP) is an index for estimating relative global warming contribution due to atmospheric emission of a kilogram of a particular greenhouse gas compared to emission of a kilogram of carbon dioxide. GWP can be calculated for different time horizons showing the effect of atmospheric lifetime for a given gas. The GWP for the 100-year time horizon is commonly the value referenced.

Throughout the definitions, the term "Cₙ₋ₘ" indicates a range which includes the endpoints, wherein n and m are integers and indicate the number of carbons. Examples include C₁₋₄, C₁₋₆, and the like.

As used herein, the term "Cₙ₋ₘ alkyl" refers to a saturated hydrocarbon group that may be straight-chain or branched, having n to m carbons. Examples of alkyl moieties include, but are not limited to, chemical groups such as methyl, ethyl, *n-*propyl, isopropyl, *n*-butyl, *tert*-butyl, isobutyl, *sec*-butyl; higher homologs such as 2-methyl-1-butyl, *n*-pentyl, 3-pentyl, *n*-hexyl, 1,2,2-trimethylpropyl, and the like. In some embodiments, the alkyl group has 1 to 20, 1 to 10, 1 to 6, 4 to 20, 4 to 12, or 4 to 8 carbon atoms.

As used herein, "halide" refers to fluoride, chloride, bromide, or iodide. In some embodiments, the halo is chloride or bromide.

As used herein, the term "alkali metal" refers to lithium, sodium, potassium, or rubidium.

As used herein, the term "quaternary (Cₙ₋ₘ alkyl)ammonium salt" refers to a salt of formula (Cₙ₋ₘ alkyl)₄N⁺X⁻, wherein X⁻ is an anionic group (*e.g*., halide, hydrogen sulfate, and the like), and each Cₙ₋ₘ alkyl refers to a saturated hydrocarbon group that may be straight-chain or branched, having n to m carbons, wherein each Cₙ₋ₘ alkyl group may be the same or different. Exemplary quaternary (Cₙ₋ₘ alkyl)ammonium salt include, but are not limited to, tetrabutylammonium chloride, tetrabutylammonium bromide, tetrabutylammonium hydrogen sulfate, tetraoctylammonium chloride, tetraoctylammonium bromide, tetraoctylammonium hydrogen sulfate, trioctylmethylammonium chloride, trioctylmethylammonium bromide, tetradecylammonium chloride, tetradecylammonium bromide, and tetradodecylammonium chloride.

As used herein the term, "hydrogenation catalyst" refers to a metal (*e.g*., palladium, nickel, or rhodium) catalyst suitable to catalyze a hydrogenation reaction (i.e., reaction of a compound with hydrogen gas). Exemplary hydrogenation catalysts include, but are not limited to, palladium on carbon, Lindlar's catalyst, Wilkinson's catalyst, HRuCl(PPh₃)₃, RhCl(PPh₃)₃, [Rh(COD)Cl]₂, [Ir(COD)(PMePh₂)₂]⁺, [Rh(1,5-cyclooctadiene)(PPh₃)₂]⁺, PtO₂ (Adam's catalyst), palladium on carbon, palladium black, a palladium catalyst, a palladium catalyst dispersed on aluminum oxide or titanium silicate doped with silver and/or a lanthanide, and the like. In some embodiments, the hydrogenation catalyst is a palladium catalyst. In some embodiments, the hydrogenation catalyst is a Lindlar's catalyst. In some embodiments, the hydrogenation catalyst is a the catalyst is a palladium catalyst dispersed on aluminum oxide or titanium silicate, doped with silver and/or a lanthanide.

### Chemicals, Abbreviations, and Acronyms

| **ID** | **Chemical Name** |
|---|---|
| R-11 or CFC-11 | trichlorofluoromethane |
| 112 | 1,1,2,2-tetrachloro-1,2-difluoroethane |
| 112a | 1,1,1,2-tetrachloro-2,2-difluoroethane |
| 113 | 1,1,2-trichloro-1,2,2-trifluoroethane |
| 114 | 1,2-dichloro-1,1,2,2-tetrafluoroethane |
| 1215xc | 2-chloro-1,1,3,3,3-pentafluoroprop-1-ene |
| 122 | 1,2,2-trichloro-1,1-difluoroethane |
| 1223xd | 1,2-dichloro-3,3,3-trifluoroprop-1-ene |
| 1224xe | 2-chloro-1,3,3,3-tetrafluoroprop-1-ene |
| 1225zc | 1,1,3,3,3-pentafluoroprop-1-ene |
| 122b | 1,1,1-trichloro-2,2-difluoroethane |
| 123 | 2,2-dichloro-1,1,1-trifluoroethane |
| 1233zd | 1 -chloro-3,3,3 -trifluoro-propene |
| 123a | 1,2-dichloro-1,1,2-trifluoroethane |
| 1314 | 1,2-dichloro-3,3,4,4-tetrafluorocvclobut-1-ene |
| 1323azd | 1,1,2,3-tetrachloro-4,4,4-trifluorobut-1-ene |
| 1325lxz | 1,2-dichloro-1,1,4,4,4-pentafluorobut-2-ene |
| 1325lze | 1-chloro-1,1,2,4,4,4-hexafluorobut-2-ene |
| 1325xd | 1,2-dichloro-3,3,4,4,4-pentafluorobut-1-ene |
| 1326lez | 1-chloro-1,1,2,4,4,4-hexafluorobut-2-ene |
| 1326mxz | 2-chloro-1,1,1,4,4,4-hexafluoro-2-butene |
| 1327mz | 1,1,1,2,4,4,4-heptafluorobut-2-ene |
| 1336mzz | 1,1,1,4,4,4-hexafluorobutene |
| 133a | 2-chloro-1,1,1-trifluoroethane |
| 225da | 1,2-dichloro-1,1,3,3,3-pentafluoropropane |
| 234da | 2,3-dichloro-1,1,1,3-tetrafluoropropane |
| 235da | 2-chloro-1,1,1,3,3-pentafluoropropane |
| 235fa | 1-chloro-1,1,3,3,3-pentafluoropropane |
| 236ea | 1,1,1,2,3,3-hexafluoropropane |
| 236fa | 1,1,1,3,3,3-hexafluoropropane |
| 244da | 2-chloro-1,1,1,3-tetrafluoropropane |
| 245fa | 1,1,1,3,3-pentafluoropropane |
| 253fa | 3-chloro-1,1,1-trifluoropropane |
| 336lbf | 1,2-dichloro-1,1,2,4,4,4-hexafluorobutane |
| 336maf | 2,2-dichloro-1,1,1,4,4,4-hexafluorobutane |
| 337mbf | 2-chloro-1,1,1,2,4,4,4-heptafluorobutane |
| 337mde | 2-chloro-1,1,1,3,4,4,4-heptafluorobutane |
| 338mf | 1,1,1,2,2,4,4,4-octafluorobutane |
| 346mdf | 2-chloro-1,1,1,4,4,4-hexafluorobutane |
| 355lff | 1-chloro-1,1,4,4,4-pentafluorobutane |
| 355mcf | 4-chloro-1,1,1,2,2-pentafluorobutane |
| 356mff | 1,1,1,4,4,4-hexafluorobutane |
| 3351dd | 1,2,3-trichloro-1,1,4,4,4-pentafluorobutane |
| dl-336mdd | dl-2,3-dichloro-1,1,1,4,4,4-hexafluorobutane |
| E-1316 | (E)-2,3-dichloro-1,1,1,4,4,4-hexafluorobut-2-ene |
| E-1326mxz | (E)-2-chloro-1,1,1,4,4,4-hexafluoro-2-butene |
| E-1336mzz | (E)-1,1,1,4,4,4-hexafluorobutene |
| *meso-*336mdd | *meso*-2,3-dichloro-1,1,1,4,4,4-hexafluorobutane |
| Z-1316 | (Z)-2,3-dichloro-1,1,1,4,4,4-hexafluorobut-2-ene |
| Z-13251xz | (Z)-1,2-dichloro-1,1,4,4,4-pentafluorobut-2-ene |
| Z-1326mxz | (Z)-2-chloro-1,1,1,4,4,4-hexafluoro-2-butene |
| Z-1336mzz | (Z)-1,1,1,4,4,4-hexafluorobutene |

### Processes

The present application provides, *inter alia,* a process of preparing 2-chloro-1,1,1,4,4,4-hexafluoro-2-butene, comprising reacting hexachlorobutadiene with hydrofluoric acid in the presence of a transition metal catalyst wherein the transition metal catalyst is a niobium catalyst selected from niobium (V) chloride, niobium (IV) chloride or a mixture thereof.

In some embodiments, greater than about 99 mole percent of the 2-chloro-1,1,1,4,4,4-hexafluoro-2-butene produced is (Z)-2-chloro-1,1,1,4,4,4-hexafluoro-2-butene.

In some embodiments, greater than about 99.5 mole percent of the 2-chloro-1,1,1,4,4,4-hexafluoro-2-butene produced by the processes provided herein is (Z)-2-chloro-1,1,1,4,4,4-hexafluoro-2-butene.

In some embodiments, greater than about 99.7 mole percent of the 2-chloro-1,1,1,4,4,4-hexafluoro-2-butene produced by the processes provided herein is (Z)-2-chloro-1,1,1,4,4,4-hexafluoro-2-butene.

In some embodiments, greater than about 99.9 mole percent of the 2-chloro-1,1,1,4,4,4-hexafluoro-2-butene produced by the processes provided herein is (Z)-2-chloro-1,1,1,4,4,4-hexafluoro-2-butene.

In some embodiments, greater than about 90 mole percent (*e.g.*, greater than about 95, greater than about 97, greater than about 99, greater than about 99.5, or greater than about 99.9 mole percent) of the hexachlorobutadiene is converted to the 2-chloro-1,1,1,4,4,4-hexafluoro-2-butene.

In some embodiments, greater than about 90 mole percent (*e.g.,* greater than about 95, greater than about 97, greater than about 99, greater than about 99.5, or greater than about 99.9 mole percent) of the hexachlorobutadiene is converted to the 2-chloro-1,1,1,4,4,4-hexafluoro-2-butene in less than about 10 hours of reacting (*e.g.*, less than about 8 hours, less than about 6 hours, less than about 5 hours of reacting).

The transition metal catalyst is a niobium halide catalyst selected from niobium (IV) chloride, niobium (V) chloride, or a mixture thereof.

The transition metal catalyst may be a mixture of tantalum chloride and niobium chloride.

In some embodiments, the transition metal catalyst is a mixture of tantalum (V) chloride and niobium (IV) chloride. In some embodiments, the transition metal catalyst is a mixture of tantalum (V) chloride and niobium (V) chloride. In some embodiments, the transition metal catalyst is a mixture of tantalum (V) chloride, niobium (IV) chloride, and niobium (V) chloride.

In some embodiments, a molar excess of hydrofluoric acid is used based on 1 molar equivalent of hexachlorobutadiene, for example, greater than 1 molar equivalent, greater than 2 molar equivalents, greater than 5 molar equivalents, greater than 10 molar equivalents, greater than 20 molar equivalents, greater than 50 molar equivalents, or greater than 100 molar equivalents of hydrofluoric acid is used based on 1 molar equivalent of hexachlorobutadiene.

In some embodiments, about 10 to about 50 molar equivalents of hydrofluoric acid is used based on 1 molar equivalent of hexachlorobutadiene, for example about 10 to about 40, about 10 to about 30, about 10 to about 20, about 20 to about 50, about 20 to about 40, about 20 to about 30, about 30 to about 50, about 30 to about 40, or about 40 to about 50 molar equivalents of hydrofluoric acid. In some embodiments, about 20 to about 30 molar equivalents of hydrofluoric acid is used based on 1 molar equivalent of hexachlorobutadiene.

In some embodiments, a catalytic amount of the catalyst is used based on 1 molar equivalent of hexachlorobutadiene, for example, less than 1 molar equivalent, less than 2 molar equivalents, less than 5 molar equivalents, less than 10 molar equivalents, less than 20 molar equivalents, less than 50 molar equivalents, or less than 100 molar equivalents of transition metal catalyst is used based on 1 molar equivalent of hexachlorobutadiene.

In some embodiments, about 0.05 to about 0.5 molar equivalents of the transition metal catalyst is used based on 1 molar equivalent of hexachlorobutadiene, for example, about 0.05 to about 0.3, about 0.05 to about 0.2, about 0.05 to about 0.1, about 0.1 to about 0.5, about 0.1 to about 0.3, about 0.1 to about 0.2, about 0.2 to about 0.5, about 0.2 to about 0.3, or about 0.3 to about 0.5 molar equivalents of transition metal catalyst is used based on 1 molar equivalent of hexachlorobutadiene. In some embodiments, about 0.1 to about 0.3 molar equivalents of the transition metal catalyst is used based on 1 molar equivalent of hexachlorobutadiene.

In some embodiments, the process is performed a temperature of from about 100°C to about 150°C, for example, about 100°C to about 140°C, about 100°C to about 130°C, about 120°C to about 140°C, about 100°C to about 110°C, about 110°C to about 150°C, about 110°C to about 140°C, about 110°C to about 130°C, about 110°C to about 140°C, about 120°C to about 150°C, about 120°C to about 140°C, about 120°C to about 130°C, about 130°C to about 150°C, about 130°C to about 140°C, or about 140°C to about 150°C. In some embodiments, the process is performed a temperature of from about 110°C to about 135°C.

In some embodiments, the process comprises:
i) adding the transition metal catalyst to the hydrofluoric acid to form a first mixture; and
ii) adding the hexachlorobutadiene to the first mixture to form a second mixture.

In some embodiments, the first mixture is heated to a temperature of from about 100°C to about 150°C, for example, about 100°C to about 140°C, about 100°C to about 130°C, about 120°C to about 140°C, about 100°C to about 110°C, about 110°C to about 150°C, about 110°C to about 140°C, about 110°C to about 130°C, about 110°C to about 140°C, about 120°C to about 150°C, about 120°C to about 140°C, about 120°C to about 130°C, about 130°C to about 150°C, about 130°C to about 140°C, or about 140°C to about 150°C. In some embodiments, the first mixture is heated to a temperature of from about 110°C to about 140°C.

In some embodiments, the process further comprises cooling the first mixture to a temperature of from about -25°C to about 25°C prior to performing step ii), for example, about -25°C to about 10°C, about -25°C to about 0°C, about -25°C to about -10°C, about -10°C to about 25°C, about -10°C to about 10°C, about -10°C to about 0°C, about 0°C to about 25°C, about 0°C to about 10°C, or about 10°C to about 25°C. In some embodiments, the process further comprises cooling the first mixture to a temperature of from about -10°C to about 10°C prior to performing step ii).

In some embodiments, the hexachlorobutadiene is added to the first mixture at a temperature of from about -25°C to about 25°C to form the second mixture, for example, about -25°C to about 10°C, about -25°C to about 0°C, about -25°C to about - 10°C, about -10°C to about 25°C, about -10°C to about 10°C, about -10°C to about 0°C, about 0°C to about 25°C, about 0°C to about 10°C, or about 10°C to about 25°C. In some embodiments, the hexachlorobutadiene is added to the first mixture at a temperature of from about -10°C to about 10°C to form the second mixture.

In some embodiments, the process further comprises heating the second mixture to a temperature of from about 100°C to about 150°C, for example, about 100°C to about 140°C, about 100°C to about 130°C, about 120°C to about 140°C, about 100°C to about 110°C, about 110°C to about 150°C, about 110°C to about 140°C, about 110°C to about 130°C, about 110°C to about 140°C, about 120°C to about 150°C, about 120°C to about 140°C, about 120°C to about 130°C, about 130°C to about 150°C, about 130°C to about 140°C, or about 140°C to about 150°C. In some embodiments, the process further comprises heating the second mixture to a temperature of from about 110°C to about 140°C.

The present application further provides a process of preparing 2-chloro-1,1,1,4,4,4-hexafluoro-2-butene, comprising:
i) adding niobium (V) chloride to hydrofluoric acid to form a first mixture;
ii) heating the first mixture to a temperature of from about 125°C to about 135°C;
iii) cooling the first mixture to a temperature of from about -10°C to about 10°C;
iv) adding hexachlorobutadiene to the first mixture to form a second mixture; and
v) heating the second mixture to a temperature of from about 125°C to about135°C.

In some embodiments, the process of preparing 2-chloro-1,1,1,4,4,4-hexafluoro-2-butene provided herein is performed as a liquid phase process. In some embodiments, the process of preparing 2-chloro-1,1,1,4,4,4-hexafluoro-2-butene (*e.g*., the liquid phase process) is performed in the absence of an additional solvent component.

In some embodiments, greater than about 99.5 mole percent of the 2-chloro-1,1,1,4,4,4-hexafluoro-2-butene produced by the processes provided herein is (Z)-2-chloro-1,1,1,4,4,4-hexafluoro-2-butene.

In some embodiments, greater than about 99.7 mole percent of the 2-chloro-1,1,1,4,4,4-hexafluoro-2-butene produced by the processes provided herein is (Z)-2-chloro-1,1,1,4,4,4-hexafluoro-2-butene.

In some embodiments, greater than about 99.9 mole percent of the 2-chloro-1,1,1,4,4,4-hexafluoro-2-butene produced by the processes provided herein is (Z)-2-chloro-1,1,1,4,4,4-hexafluoro-2-butene.

In some embodiments, the process of preparing 2-chloro-1,1,1,4,4,4-hexafluoro-2-butene further comprises reacting the 2-chloro-1,1,1,4,4,4-hexafluoro-2-butene with a base in the presence of an alkali metal halide and a quaternary (C₄₋₁₂ alkyl)ammonium salt to form perfluorobut-2-yne. In some embodiments, the process of preparing the perfluorobut-2-yne from the 2-chloro-1,1,1,4,4,4-hexafluoro-2-butene is performed according to the procedures described in U.S. Patent No. 9,328,042 and International Publication No. WO 2014/052695.

Exemplary bases include, but are not limited to, lithium hydroxide, sodium hydroxide, potassium hydroxide, calcium hydroxide, magnesium oxide, calcium oxide, sodium carbonate, potassium carbonate, sodium phosphate, potassium phosphate, and mixtures thereof. Some example strong bases include, but are not limited to, hydroxides, alkoxides, metal amides, metal hydrides, metal dialkylamides and arylamines, wherein; alkoxides include lithium, sodium and potassium salts of methyl, ethyl and t-butyl oxides; metal amides include sodium amide, potassium amide and lithium amide; metal hydrides include sodium hydride, potassium hydride and lithium hydride; and metal dialkylamides include lithium, sodium, and potassium salts of methyl, ethyl, n-propyl, *iso*-propyl, n-butyl, *tert*-butyl, trimethylsilyl and cyclohexyl substituted amides.

In some embodiments, the base is selected from the group consisting of lithium hydroxide, sodium hydroxide, potassium hydroxide, calcium hydroxide, magnesium oxide, calcium oxide, sodium carbonate, potassium carbonate, sodium phosphate, potassium phosphate, and mixtures thereof.

In some embodiments, the base is an aqueous basic solution. As used herein, the "basic aqueous solution" is a liquid (*e.g*., a solution, dispersion, emulsion, or suspension, and the like) that is primarily an aqueous liquid having a pH of over 7.

In some embodiments, the basic aqueous solution contains small amounts of organic liquids which may be miscible or immiscible with water. In some embodiments, the liquid medium in the basic aqueous solution is at least 90% water, for example, at least 95%, at least 97%, at least 98%, at least 99%, at least 99.5%, or at least 99.9%. In some embodiments, the water used in the aqueous basic solution is tap water. In some embodiments, the water is used in the aqueous basic solution deionized water or distilled water.

In some embodiments, the alkali metal halide is selected from the group consisting of lithium chloride, lithium bromide, lithium iodide, sodium chloride, sodium bromide, sodium iodide, potassium chloride, potassium bromide, potassium iodide, and mixtures thereof. In some embodiments, the alkali metal halide is sodium chloride.

In some embodiments, the quaternary (C₄₋₁₂ alkyl)ammonium salt is selected from the group consisting of tetrabutylammonium chloride, tetrabutylammonium bromide, tetrabutylammonium hydrogen sulfate, tetraoctylammonium chloride, tetraoctylammonium bromide, tetraoctylammonium hydrogen sulfate, trioctylmethylammonium chloride, trioctylmethylammonium bromide, tetradecylammonium chloride, tetradecylammonium bromide, and tetradodecylammonium chloride.

In some embodiments, the quaternary (C₄₋₁₂ alkyl)ammonium salt is a tetrabutylammonium salt. In some embodiments, the quaternary (C₄₋₁₂ alkyl)ammonium salt is selected from the group consisting of tetrabutylammonium chloride, tetrabutylammonium bromide, and tetrabutylammonium hydrogen sulfate. In some embodiments, the quaternary (C₄₋₁₂ alkyl)ammonium salt is selected from the group consisting of tetrabutylammonium halide. In some embodiments, the quaternary (C₄₋₁₂ alkyl)ammonium salt is tetrabutylammonium chloride. In some embodiments, the quaternary (C₄₋₁₂ alkyl)ammonium salt is trioctylmethylammonium chloride.

In some embodiments, about 1 to about 5 molar equivalents of base is used based on one molar equivalent of the 2-chloro-1,1,1,4,4,4-hexafluoro-2-butene, for example, about 1 to about 3, about 1 to about 2, about 1 to about 1.5, about 1.5 to about 5, about 1.5 to about 3, about 1.5 to about 2, about 2 to about 5, about 2 to about 3, or about 3 to about 5 molar equivalents. In some embodiments, about 1 to about 1.5 molar equivalents of base is used based on one molar equivalent of the 2-chloro-1,1,1,4,4,4-hexafluoro-2-butene. In some embodiments, a molar excess of base is used based on one molar equivalent of the 2-chloro-1,1,1,4,4,4-hexafluoro-2-butene.

In some embodiments, the process of preparing the perfluorobut-2-yne is performed at a temperature of from about 30°C to about 60°C, for example, about 30° to about 50°C, about 30° to about 40°C, about 40° to about 60°C, about 40° to about 50°C, or about 50°C to about 60°C.

In some embodiments, the process of preparing the perfluorobut-2-yne is performed as a liquid phase process. In some embodiments, the process of preparing the perfluorobut-2-yne is performed in the absence of an additional solvent component.

In some embodiments, the process of preparing perfluorobut-2-yne further comprises reacting the perfluorobut-2-yne with hydrogen in the presence of a hydrogenation catalyst to form 1,1,1,4,4,4-hexafluoro-2-butene. In some embodiments, the hydrogen is hydrogen gas. In some embodiments, the process of preparing the 1,1,1,4,4,4-hexafluoro-2-butene from the perfluorobut-2-yne is performed according to the procedures described in U.S. Patent No. 9,328,042 and International Publication No. WO 2014/052695.

In some embodiments, about 1 molar equivalent of hydrogen is used based on 1 molar equivalent of the perfluorobut-2-yne.

In some embodiments, about 0.5 to about 1 molar equivalents of hydrogen is used based on 1 molar equivalent of the perfluorobut-2-yne.

In some embodiments, about 0.67 to about 1 molar equivalents of hydrogen is used based on 1 molar equivalent of the perfluorobut-2-yne.

In some embodiments, the hydrogenation catalyst is a palladium catalyst. In some embodiments, the hydrogenation catalyst is Lindlar's catalyst. As used herein, the term "Lindlar's catalyst" refers to a heterogeneous palladium catalyst on a calcium carbonate support, which has been deactivated or conditioned with a lead compound. The lead compound can be, for example, lead acetate, lead oxide, or any other suitable lead compound. In some embodiments, the Lindlar's catalyst is prepared by reduction of a palladium salt in the presence of a slurry of calcium carbonate, followed by the addition of the lead compound. In some embodiments, the palladium salt is palladium chloride. In some embodiments, the catalyst is deactivated or conditioned with quinoline.

In some embodiments, the hydrogenation catalyst is a palladium catalyst dispersed on aluminum oxide or titanium silicate, doped with silver and/or a lanthanide. In some embodiments, the palladium loading on the aluminum oxide or titanium silicate is from 100 ppm to 5000 ppm. In some embodiments, the palladium loading on the aluminum oxide or titanium silicate is from 200 ppm to 5000 ppm.

In some embodiments, the hydrogenation catalyst is doped with at least one of silver, cerium, or lanthanum.

In some embodiments, the hydrogenation catalyst is doped with silver. In some embodiments the molar ratio of silver to palladium is about 0.5:1.0.

In some embodiments, the hydrogenation catalyst is doped with cerium or lanthanum. In some embodiments, the molar ratio of cerium or lanthanum to palladium is from about 2:1 to about 3:1.

In some embodiments, a catalytic amount (*i*.*e*., less than 1 molar equivalent) of the hydrogenation catalyst is used based on 1 molar equivalent of the perfluorobut-2-yne.

In some embodiments, about 0.5 to about 4 percent by weight of the hydrogenation catalyst is used based on the weight of perfluorobut-2-yne.

In some embodiments, about 1 to about 3 percent by weight of the hydrogenation catalyst is used based on the weight of perfluorobut-2-yne.

In some embodiments, about 1 to about 2 percent by weight of the hydrogenation catalyst is used based on the weight of perfluorobut-2-yne.

In some embodiments, the process of preparing the 1,1,1,4,4,4-hexafluoro-2-butene is performed at about room temperature.

In some embodiments, the process of preparing the 1,1,1,4,4,4-hexafluoro-2-butene is performed at a temperature greater than room temperature.

In some embodiments, the process of preparing the 1,1,1,4,4,4-hexafluoro-2-butene is performed at a temperature of from about 40°C to about 90°C.

In some embodiments, the process of preparing the 1,1,1,4,4,4-hexafluoro-2-butene is performed at a temperature of from about 60°C to about 90°C.

In some embodiments, the process of preparing the 1,1,1,4,4,4-hexafluoro-2-butene is performed at a temperature less than room temperature.

In some embodiments, the process of preparing the 1,1,1,4,4,4-hexafluoro-2-butene is performed at a temperature less than about 0°C.

In some embodiments, the process of preparing the 1,1,1,4,4,4-hexafluoro-2-butene is performed at a temperature of from about -70°C to about -80°C.

In some embodiments, greater than about 95 mole percent of the 1,1,1,4,4,4-hexafluoro-2-butene produced by the process provided herein is (Z)-1,1,1,4,4,4-hexafluoro-2-butene, for example, greater than about 97 mole percent, greater than about 98 mole percent, greater than about 99 mole percent, greater than about 99.5 mole percent, greater than about 99.9 mole percent. In some embodiments, greater than about 99 mole percent of the 1,1,1,4,4,4-hexafluoro-2-butene produced by the process provided herein is (Z)-1,1,1,4,4,4-hexafluoro-2-butene.

In some embodiments, the process of preparing the 1,1,1,4,4,4-hexafluoro-2-butene is performed as a liquid phase process. In some embodiments, the process of preparing the 1,1,1,4,4,4-hexafluoro-2-butene is performed in the absence of an additional solvent component.

The present application further provides a process of preparing (Z)-1,1,1,4,4,4-hexafluoro-2-butene, comprising:
i) reacting hexachlorobutadiene with hydrofluoric acid in the presence of niobium (V) chloride to form 2-chloro-1,1,1,4,4,4-hexafluoro-2-butene, wherein greater than about 99 mole percent of the 2-chloro-1,1,1,4,4,4-hexafluoro-2-butene produced is (Z)-2-chloro-1,1,1,4,4,4-hexafluoro-2-butene;
ii) reacting the (Z)-2-chloro-1,1,1,4,4,4-hexafluoro-2-butene with sodium hydroxide in the presence of sodium chloride and trioctylmethylammonium salt to form perfluorobut-2-yne; and
iii) reacting the perfluorobut-2-yne with hydrogen in the presence of a hydrogenation catalyst to form the (Z)-1,1,1,4,4,4-hexafluoro-2-butene.

The present application further provides a process of preparing a composition comprising (Z)-1,1,1,4,4,4-hexafluoro-2-butene, comprising:
i) reacting hexachlorobutadiene with hydrofluoric acid in the presence of niobium (V) chloride to form a first composition comprising 2-chloro-1,1,1,4,4,4-hexafluoro-2-butene, wherein greater than about 99 mole percent of the 2-chloro-1,1,1,4,4,4-hexafluoro-2-butene produced is (Z)-2-chloro-1,1,1,4,4,4-hexafluoro-2-butene;
ii) reacting the first composition with sodium hydroxide in the presence of sodium chloride and trioctylmethylammonium salt to form a second composition comprising perfluorobut-2-yne; and
iii) reacting the second composition in the presence of a hydrogenation catalyst to form the composition comprising (Z)-1,1,1,4,4,4-hexafluoro-2-butene.

In some embodiments, the first composition comprises greater than about 95 mole percent (Z)-2-chloro-1,1,1,4,4,4-hexafluoro-2-butene. In some embodiments, the first composition comprises greater than about 97 mole percent (Z)-2-chloro-1,1,1,4,4,4-hexafluoro-2-butene. In some embodiments, the first composition comprises greater than about 98 mole percent (Z)-2-chloro-1,1,1,4,4,4-hexafluoro-2-butene. In some embodiments, the first composition comprises greater than about 99 mole percent (Z)-2-chloro-1,1,1,4,4,4-hexafluoro-2-butene. In some embodiments, the first composition comprises greater than about 99.5 mole percent (Z)-2-chloro-1,1,1,4,4,4-hexafluoro-2-butene. In some embodiments, the first composition comprises greater than about 99.9 mole percent (Z)-2-chloro-1,1,1,4,4,4-hexafluoro-2-butene.

In some embodiments, the second composition comprises greater than about 95 mole percent perfluorobut-2-yne. In some embodiments, the second composition comprises greater than about 97 mole percent perfluorobut-2-yne. In some embodiments, the second composition comprises greater than about 98 mole percent perfluorobut-2-yne. In some embodiments, the second composition comprises greater than about 99 mole percent perfluorobut-2-yne. In some embodiments, the second composition comprises greater than about 99.5 mole percent perfluorobut-2-yne. In some embodiments, the second composition comprises greater than about 99.9 mole percent perfluorobut-2-yne.

In some embodiments, the composition comprising (Z)-1,1,1,4,4,4-hexafluoro-2-butene comprises greater than about 95 mole percent (Z)-1,1,1,4,4,4-hexafluoro-2-butene. In some embodiments, the composition comprising (Z)-1,1,1,4,4,4-hexafluoro-2-butene comprises greater than about 97 mole percent (Z)-1,1,1,4,4,4-hexafluoro-2-butene. In some embodiments, the composition comprising (Z)-1,1,1,4,4,4-hexafluoro-2-butene comprises greater than about 98 mole percent (Z)-1,1,1,4,4,4-hexafluoro-2-butene. In some embodiments, the composition comprising (Z)-1,1,1,4,4,4-hexafluoro-2-butene comprises greater than about 99 mole percent (Z)-1,1,1,4,4,4-hexafluoro-2-butene. In some embodiments, the composition comprising (Z)-1,1,1,4,4,4-hexafluoro-2-butene comprises greater than about 99.5 mole percent (Z)-1,1,1,4,4,4-hexafluoro-2-butene. In some embodiments, the composition comprising (Z)-1,1,1,4,4,4-hexafluoro-2-butene comprises greater than about 99.9 mole percent (Z)-1,1,1,4,4,4-hexafluoro-2-butene.

### Compositions

The present application further describes compositions comprising a major component (e.g., (Z)-2-chloro-1,1,1,4,4,4-hexafluoro-2-butene, or perfluorobut-2-yne, or (Z)-1,1,1,4,4,4-hexafluoro-2-butene) in combination with one or more additional compounds. The compositions may be prepared according to one or more of the processes described herein. The compositions do not form part of the invention for which protection is sought.

The additional compounds of the compositions described herein may provide improved solubility for active ingredients in an aerosol or polymer constituents of a foam. Additionally, for refrigerant applications, such as use in air conditioning, heat pumps, refrigeration, and power cycles (e.g., organic Rankine cycles), the additional compounds may provide improved solubility with refrigeration lubricants, such as mineral oils, alkylbenzenes, synthetic paraffins, synthetic naphthenes, poly(alpha)olefins, polyol esters (POE), polyalkylene glycols (PAG), polyvinyl ethers (PVE), or perfluoropolyethers (PFPE), or mixtures thereof.

Further, the presence of the additional compounds in a sample of (Z)-2-chloro-1,1,1,4,4,4-hexafluoro-2-butene, or perfluorobut-2-yne, or (Z)-1,1,1,4,4,4-hexafluoro-2-butene may be used to identify the process by which the compound was manufactured.

### (Z)-2-chloro-1,1,1,4,4,4-hexafluoro-2-butene

The present application further provides a composition, comprising:
i) (Z)-2-chloro-1,1,1,4,4,4-hexafluoro-2-butene; and
ii) one or more additional compounds selected from the group consisting of:
   1,1,1,3,3,3-hexafluoropropane;
   1,1,1,2,2,4,4,4-octafluorobutane;
   1,1,1,4,4,4-hexafluorobutane;
   1,2-dichloro-1,1,2,2-tetrafluoroethane;
   2-chloro-1,1,1 -trifluoroethane;
   2-chloro-1,1,1,2,4,4,4-heptafluorobutane;
   2-chloro-1,1,1,3,4,4,4-heptafluorobutane;
   2-chloro-1,1,1,3,3-pentafluoropropane;
   1-chloro-1,1,3,3 ,3-pentafluoropropane;
   (E)-2-chloro-1,1,1,4,4,4-hexafluorobut-2-ene;
   1,2-dichloro-3,3,4,4-tetrafluorocyclobut-1-ene;
   2-chloro-1,1,1,4,4,4-hexafluorobutane;
   2,2-dichloro-1,1,1-trifluoroethane;
   1,2-dichloro-1,1,2-trifluoroethane;
   1,2-dichloro-1,1,3,3,3-pentafluoropropane;
   (E)-2,3-dichloro-1,1,1,4,4,4-hexafluorobut-2-ene;
   1,1,2-trichloro-1,2,2-trifluoroethane;
   (Z)-2,3-dichloro-1,1,1,4,4,4-hexafluorobut-2-ene;
   1,2-dichloro-3,3,3-trifluoroprop-1-ene;
   (Z)-1,2-dichloro-1,1,4,4,4-pentafluorobut-2-ene;
   2,2-dichloro-1,1,1,4,4,4-hexafluorobutane;
   dl-2,3-dichloro-1,1,1,4,4,4-hexafluorobutane;
   *meso*-2,3-dichloro-1,1,1,4,4,4-hexafluorobutane;
   1,2-dichloro-3,3,4,4,4-pentafluorobut-1-ene;
   2,3-dichloro-1,1,1,3-tetrafluoropropane;
   1,2-dichloro-1,1,2,4,4,4-hexafluorobutane;
   1,2,2-trichloro-1,1-difluoroethane;
   1,1,1 -trichloro-2,2-difluoroethane;
   1,1,2,2-tetrachloro-1,2-difluoroethane;
   1,1,1,2-tetrachloro-2,2-difluoroethane;
   1,2,3-trichloro-1,1,4,4,4-pentafluorobutane; and
   1,1,2,3-tetrachloro-4,4,4-trifluorobut-1-ene;
   wherein the composition comprises greater than about 95 mole percent (Z)-2-chloro-1,1,1,4,4,4-hexafluoro-2-butene.

The composition described herein may comprise (Z)-2-chloro-1,1,1,4,4,4-hexafluoro-2-butene and one of the additional compounds. The composition described herein may comprise (Z)-2-chloro-1,1,1,4,4,4-hexafluoro-2-butene and more than one of the additional compounds (e.g., two or more; three or more; five or more; ten or more; and the like).

The composition described herein may comprise (Z)-2-chloro-1,1,1,4,4,4-hexafluoro-2-butene and each of the additional compounds. The composition described herein may comprise (Z)-2-chloro-1,1,1,4,4,4-hexafluoro-2-butene and from one to twenty-five of the additional compounds. The composition described herein may comprise (Z)-2-chloro-1,1,1,4,4,4-hexafluoro-2-butene and from one to twenty of the additional compounds. The composition described herein may comprise (Z)-2-chloro-1,1,1,4,4,4-hexafluoro-2-butene and from one to ten of the additional compounds. The composition described herein may comprise (Z)-2-chloro-1,1,1,4,4,4-hexafluoro-2-butene and from one to five of the additional compounds. The composition described herein may comprise (Z)-2-chloro-1,1,1,4,4,4-hexafluoro-2-butene and from one to four of the additional compounds. The composition described herein may comprise (Z)-2-chloro-1,1,1,4,4,4-hexafluoro-2-butene and from one to three of the additional compounds. The composition described herein may comprise (Z)-2-chloro-1,1,1,4,4,4-hexafluoro-2-butene and from one to two of the additional compounds.

The composition described herein may comprise:
i) (Z)-2-chloro-1,1,1,4,4,4-hexafluoro-2-butene; and
ii) one or more additional compounds selected from the group consisting of:
   2-chloro-1,1,1,2,4,4,4-heptafluorobutane;
   (E)-2-chloro-1,1,1,4,4,4-hexafluorobut-2-ene;
   1,2-dichloro-3,3,4,4-tetrafluorocyclobut-1-ene;
   1,2-dichloro-1,1,3,3,3-pentafluoropropane; and
   1,1,2-trichloro-1,2,2-trifluoroethane;
   wherein the composition comprises greater than about 95 mole percent (Z)-2-chloro-1,1,1,4,4,4-hexafluoro-2-butene.

The composition described herein may comprise greater than about 97 mole percent (Z)-2-chloro-1,1,1,4,4,4-hexafluoro-2-butene. The composition described herein may comprise greater than about 98 mole percent (Z)-2-chloro-1,1,1,4,4,4-hexafluoro-2-butene. The composition described herein may comprise greater than about 99 mole percent (Z)-2-chloro-1,1,1,4,4,4-hexafluoro-2-butene. The composition described herein may comprise greater than about 99.5 mole percent (Z)-2-chloro-1,1,1,4,4,4-hexafluoro-2-butene. The composition described herein may comprise greater than about 99.9 mole percent (Z)-2-chloro-1,1,1,4,4,4-hexafluoro-2-butene.

The composition described herein may consist essentially of the (Z)-2-chloro-1,1,1,4,4,4-hexafluoro-2-butene and the one or more additional compounds.

### Perfluorobut-2-yne

The present application further describes a composition, comprising:
i) perfluorobut-2-yne; and
ii) one or more additional compounds selected from the group consisting of:
   1,1,1,3,3,3-hexafluoropropane;
   1,1,1,2,4,4,4-heptafluorobut-2-ene;
   (E)-1,1,1,4,4,4-hexafluorobutene;
   1,1,1,2,2,4,4,4-octafluorobutane;
   1,1,1,4,4,4-hexafluorobutane;
   1,2-dichloro-1,1,2,2-tetrafluoroethane;
   2-chloro-1,1,1 -trifluoroethane;
   (Z)-2-chloro-1,1,1,4,4,4-hexafluorobut-2-ene;
   1-chloro-3,3,4,4,4-pentafluotobut-1-yne;
   1-chloro-3,3,4,4,4-pentafluorobut-2-yne;
   (Z)-1,2-dichloro-1,1,4,4,4-pentafluorobut-2-ene;
   1,2-dichloro-3,3,4,4,4-pentafluorobut-1-ene
   2-chloro-1,1,1,2,4,4,4-heptafluorobutane;
   2-chloro-1,1,1,3,4,4,4-heptafluorobutane;
   2-chloro-1,1,1,3,3-pentafluoropropane;
   1-chloro-1,1,3,3,3-pentafluoropropane;
   (E)-2-chloro-1,1,1,4,4,4-hexafluorobut-2-ene;
   2-chloro-1,1,1,4,4,4-hexafluorobutane;
   2,2-dichloro-1,1,1-trifluoroethane;
   1,2-dichloro-1,1,2-trifluoroethane;
   1,2-dichloro-1,1,3,3,3-pentafluoropropane;
   (E)-2,3-dichloro-1,1,1,4,4,4-hexafluorobut-2-ene;
   1,1,2-trichloro-1,2,2-trifluoroethane;
   (Z)-2,3-dichloro-1,1,1,4,4,4-hexafluorobut-2-ene;
   1-chloro-3,3,3-trifluoroprop-1-yne;
   1,2-dichloro-3,3,3-trifluoroprop-1-ene;
   (Z)-1,2-dichloro-1,1,4,4,4-pentafluorobut-2-ene;
   1-chloro-1,1,2,4,4,4-hexafluorobut-2-ene;
   2-chloro-1,3,3,3 -tetrafluoroprop-1 -ene;
   1,1,3,3,3-pentafluoroprop-1-ene; and
   2-chloro-1,1,3,3,3-pentafluoroprop-1-ene;
   wherein the composition comprises greater than about 95 mole percent perfluorobut-2-yne.

The composition described herein may comprise perfluorobut-2-yne and one of the additional compounds. The composition described herein may comprise perfluorobut-2-yne and more than one of the additional compounds (*e.g*., two or more; three or more; five or more; ten or more; and the like).

The composition described herein may comprise perfluorobut-2-yne and each of the additional compounds. The composition described herein may comprise perfluorobut-2-yne and from one to twenty-five of the additional compounds. The composition described herein may comprise perfluorobut-2-yne and from one to twenty of the additional compounds. The composition described herein may comprise perfluorobut-2-yne and from one to ten of the additional compounds. The composition described herein may comprise perfluorobut-2-yne and from one to five of the additional compounds. The composition described herein may comprise perfluorobut-2-yne and from one to four of the additional compounds. The composition described herein may comprise perfluorobut-2-yne and from one to three of the additional compounds. The composition described herein may comprise perfluorobut-2-yne and from one to two of the additional compounds.

The composition described herein may comprise
i) perfluorobut-2-yne; and
ii) one or more additional compounds selected from the group consisting of:
   2-chloro-1,1,1,2,4,4,4-heptafluorobutane;
   (E)-2-chloro-1,1,1,4,4,4-hexafluorobut-2-ene; and
   1,1,2-trichloro-1,2,2-trifluoroethane;
wherein the composition comprises greater than about 95 mole percent perfluorobut-2-yne.

The composition described herein may comprise greater than about 97 mole percent perfluorobut-2-yne. The composition described herein may comprise greater than about 98 mole percent perfluorobut-2-yne. The composition described herein may comprise greater than about 99 mole percent perfluorobut-2-yne. The composition described herein may comprise greater than about 99.5 mole percent perfluorobut-2-yne. The composition described herein may comprise greater than about 99.9 mole percent perfluorobut-2-yne.

The composition described herein may consist essentially of the perfluorobut-2-yne and the one or more additional compounds.

### (Z)-1,1,1,4,4,4-hexafluoro-2-butene

The present application further describes a composition, comprising:
i) (Z)-1,1,1,4,4,4-hexafluoro-2-butene; and
ii) one or more additional compounds selected from the group consisting of:
   1,1,1,3,3,3-hexafluoropropane;
   (E)-1,1,1,4,4,4-hexafluorobutene;
   1,1,1,2,2,4,4,4-octafluorobutane;
   1,2-dichloro-1,1,2,2-tetrafluoroethane;
   3-chloro-1,1,1-trifluoropropane;
   4-chloro-1,1,1,2,2-pentafluorobutane;
   2-chloro-1,1,1,2,4,4,4-heptafluorobutane;
   2-chloro-1,1,1,3,4,4,4-heptafluorobutane;
   2-chloro-1,1,1,3,3-pentafluoropropane;
   1-chloro-1,1,3,3 ,3-pentafluoropropane;
   2-chloro-1,1,1,4,4,4-hexafluorobutane;
   1,2-dichloro-1,1,3,3,3-pentafluoropropane;
   1,2-dichloro-3,3,3-trifluoroprop-1-ene;
   1-chloro-3,3,3-trifluoro-propene;
   1-chloro-1,1,2,4,4,4-hexafluorobut-2-ene;
   1-chloro-1,1,4,4,4-pentafluorobutane;
   2-chloro-1,1,1,3-tetrafluoropropane; and
   1,1,1,3,3-pentafluoropropane;
   wherein the composition comprises greater than about 99 mole percent (Z)-1,1,1,4,4,4-hexafluoro-2-butene.

The composition described herein may comprise (Z)-1,1,1,4,4,4-hexafluoro-2-butene and one of the additional compounds. The composition described herein may comprise (Z)-1,1,1,4,4,4-hexafluoro-2-butene and more than one of the additional compounds (*e.g*., two or more; three or more; five or more; ten or more; and the like).

The composition described herein may comprise (Z)-1,1,1,4,4,4-hexafluoro-2-butene and each of the additional compounds. The composition described herein may comprise (Z)-1,1,1,4,4,4-hexafluoro-2-butene and from one to ten of the additional compounds. The composition described herein may comprise (Z)-1,1,1,4,4,4-hexafluoro-2-butene and from one to five of the additional compounds. The composition described herein may comprise (Z)-1,1,1,4,4,4-hexafluoro-2-butene and from one to four of the additional compounds. The composition described herein may comprise (Z)-1,1,1,4,4,4-hexafluoro-2-butene and from one to three of the additional compounds. The composition described herein may comprise (Z)-1,1,1,4,4,4-hexafluoro-2-butene and from one to two of the additional compounds.

The composition described herein may comprise:
i) (Z)-1,1,1,4,4,4-hexafluoro-2-butene; and
ii) one or more additional compounds selected from the group consisting of:
   2-chloro-1,1,1,2,4,4,4-heptafluorobutane; and
   1,2-dichloro-1,1,3,3,3-pentafluoropropane;
   wherein the composition comprises greater than about 99 mole percent (Z)-1,1,1,4,4,4-hexafluoro-2-butene.

The composition described herein may comprise greater than about 97 mole percent (Z)-1,1,1,4,4,4-hexafluoro-2-butene. The composition described herein may comprise greater than about 98 mole percent (Z)-1,1,1,4,4,4-hexafluoro-2-butene. The composition described herein may comprise greater than about 99 mole percent (Z)-1,1,1,4,4,4-hexafluoro-2-butene. The composition described herein may comprise greater than about 99.5 mole percent (Z)-1,1,1,4,4,4-hexafluoro-2-butene. The composition described herein may comprise greater than about 99.9 mole percent (Z)-1,1,1,4,4,4-hexafluoro-2-butene.

The composition described herein may consist essentially of the (Z)-1,1,1,4,4,4-hexafluoro-2-butene and the one or more additional compounds.

### Methods of Use

The composition described herein may be useful, for example, in a wide range of applications, including their use as refrigerants, uses in high-temperature heat pumps, organic Rankine cycles, as fire extinguishing/fire suppression agents, propellants, foam blowing agents, solvents, and/or cleaning fluids. The methods of use do not form part of the invention for which protection is sought.

The additional compounds of the compositions described herein containing at least one chlorine atom may provide improved solubility for the major component of the composition (*e.g*., (Z)-2-chloro-1,1,1,4,4,4-hexafluoro-2-butene, or perfluorobut-2-yne, or (Z)-1,1,1,4,4,4-hexafluoro-2-butene) in an aerosol or polymer constituents of a foam.

For example, unsaturated fluorocarbons, such as (Z)-1,1,1,4,4,4-hexafluoro-2-butene, exhibit different solubility than other fluorocarbon propellants. This reduced solubility can make it difficult to prepare single phase aqueous homogenous aerosol formulations. The presence of low level chlorinated impurities can improve mixing and ease formulations and use of aerosol products.

Unsaturated fluorocarbons, such as (Z)-2-chloro-1,1,1,4,4,4-hexafluoro-2-butene, also exhibit different solubility than other common blowing agents. The reduced solubility can assist in seeding small cell growth during a foaming reaction but the compounds can be difficult to mix. The presence of low level chlorinated impurities can improve mixing and foam processing performance without sacrificing the benefits from the lower HFO solubility. Also, the chlorinated compounds typically have lower vapor thermal conductivities and so will impart improved insulating performance to a foam insulation product.

Additionally, for refrigerant applications, such as use in air conditioning, heat pumps, refrigeration, and power cycles (*e.g*., organic Rankine cycles), additional compounds containing at least one chlorine atom may provide improved solubility with refrigeration lubricants, such as mineral oils, alkylbenzenes, synthetic paraffins, synthetic naphthenes, poly(alpha)olefins, polyol esters (POE), polyalkylene glycols (PAG), polyvinyl ethers (PVE), or perfluoropolyethers (PFPE) or mixtures thereof.

Further, the additional compounds of the compositions described herein may assist in improving leak detection ability. Leakage of refrigerants may lead to loss of refrigerant from a system, thus increasing cost of operation due to the need to top-off refrigerant charge, and even minor loss of refrigerant from a system may impact proper operation. Finally, leakage of refrigerant may lead to excessive environmental contamination. In particular, chlorinated compounds, even at low levels can increase the detectability of refrigerant at the point of a leak. Thus, the system may be repaired or redesigned to prevent refrigerant leakage.

The levels of additional compounds (*e.g*., additional chlorinated compounds) must be kept low, however, because higher levels of the additional compounds may create compatibility problems with materials of construction. In aerosols, these compatibility problems may be with the aerosol container (*e.g*., cans) or with plastic valve parts. In foams, these compatibility problems may be with equipment seals and gaskets. Additionally, in aerosol products interaction of higher levels of additional compounds (*e.g*., chlorinated compounds) may cause instability of the formulation. For example, in foam products, higher levels of chlorinated compounds may soften the foam resulting in dimensional instability and poor strength of the foam.

The compositions described herein may also useful as low global warming potential (GWP) heat transfer compositions, refrigerants, power cycle working fluids, aerosol propellants, foaming agents, blowing agents, solvents, cleaning agents, carrier fluids, displacement drying agents, buffing abrasion agents, polymerization media, expansion agents for poly-olefins and polyurethane, gaseous dielectrics, fire extinguishing agents, and fire suppression agents, in liquid or gaseous form. The compositions described herein may be useful as a working fluid used to carry heat from a heat source to a heat sink. Such heat transfer compositions may also be useful as a refrigerant in a cycle wherein the fluid undergoes a phase change (e.g., from a liquid to a gas and back or vice versa).

Examples of heat transfer systems include but are not limited to air conditioners, freezers, refrigerators, heat pumps, water chillers, flooded evaporator chillers, direct expansion chillers, walk-in coolers, heat pumps, mobile refrigerators, mobile air conditioning units and combinations thereof.

The compositions described herein may be useful in mobile heat transfer systems, including refrigeration, air conditioning, or heat pump systems or apparatus. The compositions may be useful in stationary heat transfer systems, including refrigeration, air conditioning, or heat pump systems or apparatus.

As used herein, mobile heat transfer systems refers to any refrigeration, air conditioner, or heating apparatus incorporated into a transportation unit for the road, rail, sea or air. In addition, mobile refrigeration or air conditioner units, include those apparatus that are independent of any moving carrier and are known as "intermodal" systems. Such intermodal systems include "containers' (combined sealland transport) as well as "swap bodies" (combined road/rail transport).

As used herein, stationary heat transfer systems are systems that are fixed in place during operation. A stationary heat transfer system may be associated within or attached to buildings of any variety or may be stand-alone devices located out of doors, such as a soft drink vending machine. These stationary applications may be stationary air conditioning and heat pumps (including but not limited to chillers, high temperature heat pumps, including trans-critical heat pumps (*e.g*., with condenser temperatures above 50°C, above 70°C, above 80°C, above 100°C, above 120°C, above 140°C, above 160°C, above 180°C, or above 200°C), residential, commercial or industrial air conditioning systems, and including window, ductless, ducted, packaged terminal, chillers, and those exterior but connected to the building such as rooftop systems). In stationary refrigeration applications, the compositions provided herein may be useful in high temperature, medium temperature, and/or low temperature refrigeration equipment including commercial, industrial or residential refrigerators and freezers, ice machines, self-contained coolers and freezers, flooded evaporator chillers, direct expansion chillers, walk-in and reach-in coolers and freezers, and combination systems. The described compositions may be used in supermarket refrigerator systems.

Therefore, compositions described herein may be useful in methods for producing cooling, producing heating, and transferring heat.

The present application further describes a method for producing cooling comprising evaporating a composition described herein in the vicinity of a body to be cooled, and thereafter condensing said composition.

The present application further describes a method for producing heating comprising condensing a composition described herein in the vicinity of a body to be heated, and thereafter evaporating said compositions.

The present application further describes a method of using compositions described herein as heat transfer fluid compositions. The method may comprise transporting said composition from a heat source to a heat sink.

The compositions described herein may also be useful as low global warming potential (GWP) replacements for currently used refrigerants, including but not limited to, R-123 (*i*.*e*., HFC-123, 2,2-dichloro-1,1,1-trifluoroethane), R-11 (*i*.*e*., CFC-11, trichlorofluoromethane), R-245fa (*i.e.* HFC-245fa, 1,1,1,3,3-pentafluoropropane), R-114 (*i*.*e*., CFC-114, 1,2-dichloro-1,1,2,2-tetrafluoroethane), R-236fa (*i.e.,* HFC-236a, 1,1,1,3,3,3-hexafluoropropane), R-236ea (*i.e*., HFC-236ea, 1,1,1,2,3,3-hexafluoropropane), R-124 (*i*.*e*., HCFC-124, 2-chloro-1,1,1,2-tetrafluoroethane), among others.

The composition described herein may be useful as refrigerants and provide at least comparable cooling performance (*i*.*e*., cooling capacity and energy efficiency) as the refrigerant for which a replacement is being sought. Additionally, the compositions described herein may provide heating performance (*i*.*e*., heating capacity and energy efficiency) comparable to a refrigerant being replaced.

The present application further describes a method for recharging a heat transfer system that contains a refrigerant to be replaced and a lubricant, said method comprising removing the refrigerant to be replaced from the heat transfer system while retaining a substantial portion of the lubricant in said system and introducing one of the compositions described herein to the heat transfer system. The lubricant in the system may be partially replaced (*e.g*., replace a portion of the mineral oil lubricant used with HCFC-123 with a POE lubricant).

The compositions described herein may be used to top-off a refrigerant charge in a chiller. For example, if a chiller using HCFC-123 has diminished performance due to leakage of refrigerant, the compositions provided herein may be added to bring performance back up to specification.

The present application further describes a heat exchange system containing any of the compositions described herein, wherein said system is selected from the group consisting of air conditioners, freezers, refrigerators, heat pumps, water chillers, flooded evaporator chillers, direct expansion chillers, walk-in coolers, heat pumps, mobile refrigerators, mobile air conditioning units, and systems having combinations thereof. Additionally, the compositions described herein may be useful in secondary loop systems wherein these compositions serve as the primary refrigerant thus providing cooling to a secondary heat transfer fluid that thereby cools a remote location.

Vapor-compression refrigeration, air-conditioning, or heat pump systems include an evaporator, a compressor, a condenser, and an expansion device. A vapor-compression cycle re-uses refrigerant in multiple steps producing a cooling effect in one step and a heating effect in a different step. The cycle can be described simply as follows: Liquid refrigerant enters an evaporator through an expansion device, and the liquid refrigerant boils in the evaporator, by withdrawing heat from the environment, at a low temperature to form a vapor and produce cooling. The low-pressure vapor enters a compressor where the vapor is compressed to raise its pressure and temperature. The higher-pressure (compressed) vapor refrigerant then enters the condenser in which the refrigerant condenses and discharges its heat to the environment. The refrigerant returns to the expansion device through which the liquid expands from the higher-pressure level in the condenser to the low-pressure level in the evaporator, thus repeating the cycle.

The present application further describes foam expansion agent compositions comprising a composition described herein for use in preparing foams. The present application further describes foamable compositions, including but not limited to, thermoset (*e.g*., polyurethane, polyisocyanurate, or phenolic) foam compositions, thermoplastic (*e.g*., polystyrene, polyethylene, or polypropylene) foam compositions and methods of preparing foams. One or more of the compositions described herein may be included as a foam expansion agent in the foamable compositions, wherein foamable composition may include one or more additional components capable of reacting and/or mixing and foaming under the proper conditions to form a foam or cellular structure.

The present application further describes a method of forming a foam comprising: (a) adding to a foamable composition a composition described herein; and (b) processing the foamable composition under conditions effective to form a foam.

The present application further describes the use of the compositions described herein as propellants in sprayable compositions. Additionally, the present application further describes sprayable compositions. The active ingredient to be sprayed together with inert ingredients, solvents, and other materials may also be present in a sprayable composition. The sprayable composition may be an aerosol. The compositions described herein can also be used to formulate a variety of industrial aerosols or other sprayable compositions such as contact cleaners, dusters, lubricant sprays, mold release sprays, insecticides, and the like, and consumer aerosols such as personal care products (*e.g.,* hair sprays, deodorants, and perfumes), household products (*e.g.*, waxes, polishes, pan sprays, room fresheners, and household insecticides), and automotive products (*e.g*., cleaners and polishers), as well as medicinal materials such as anti-asthma and anti-halitosis medications. Examples include, but are not limited to, metered dose inhalers (MDIs) for the treatment of asthma and other chronic obstructive pulmonary diseases and for delivery of medicaments to accessible mucous membranes or intra-nasally.

Further described is a process for producing aerosol products comprising the step of adding a composition described herein to a formulation to an aerosol container, wherein said composition functions as a propellant. Additionally, the present application further describes a process for producing aerosol products comprising the step of adding a composition described herein to a barrier type aerosol package (*e*.*g*.*,* a bag-in-a-can or piston can) wherein said composition is kept separated from other formulation ingredients in an aerosol container, and wherein said composition functions as a propellant. Additionally, the present application further describes a process for producing aerosol products comprising the step of adding only a composition described herein to an aerosol package, wherein said composition functions as the active ingredient (*e.g*., a duster, or a cooling or freezing spray).

The present application further describes a process for converting heat from a heat source to mechanical energy, comprising heating a working fluid comprising a composition described herein and thereafter expanding the heated working fluid. In the process, heating of the working fluid uses heat supplied from the heat source; and expanding of the heated working fluid generates mechanical energy as the pressure of the working fluid is lowered.

The process for converting heat may be a subcritical cycle, a trans-critical cycle, or a supercritical cycle. In a transcritical cycle, the working fluid is compressed to a pressure above its critical pressure prior to being heated, and then during expansion the working fluid pressure is reduced to below its critical pressure. In a super critical cycle, the working fluid remains above its critical pressure for the complete cycle (*e.g*., compression, heating, expansion and cooling).

Heat sources may include, for example, low pressure steam, industrial waste heat, solar energy, geothermal hot water, low-pressure geothermal steam (primary or secondary arrangements), or distributed power generation equipment utilizing fuel cells or prime movers such as turbines, microturbines, or internal combustion engines. One source of low-pressure steam could be the process known as a binary geothermal Rankine cycle. Large quantities of low-pressure steam can be found in numerous locations, such as in fossil fuel powered electrical generating power plants. Other sources of heat include waste heat recovered from gases exhausted from mobile internal combustion engines (*e.g*., truck or rail diesel engines or ships), waste heat from exhaust gases from stationary internal combustion engines (*e.g*., stationary diesel engine power generators), waste heat from fuel cells, heat available at combined heating, cooling and power or district heating and cooling plants, waste heat from biomass fueled engines, heat from natural gas or methane gas burners or methane-fired boilers or methane fuel cells (*e.g*., at distributed power generation facilities) operated with methane from various sources including biogas, landfill gas and coal-bed methane, heat from combustion of bark and lignin at paper/pulp mills, heat from incinerators, heat from low pressure steam at conventional steam power plants (to drive "bottoming" Rankine cycles), and geothermal heat.

The process of converting heat may be performed using an organic Rankine power cycle. Heat available at relatively low temperatures compared to steam (inorganic) power cycles can be used to generate mechanical power through Rankine cycles using working fluids as described herein. The working fluid may be compressed prior to being heated. Compression may be provided by a pump which pumps working fluid to a heat transfer unit (*e.g*., a heat exchanger or an evaporator) where heat from the heat source is used to heat the working fluid. The heated working fluid is then expanded, lowering its pressure. Mechanical energy is generated during the working fluid expansion using an expander. Examples of expanders include, but are not limited to, turbo or dynamic expanders, such as turbines, and positive displacement expanders, such as screw expanders, scroll expanders, and piston expanders. Examples of expanders also include rotary vane expanders.

Mechanical power can be used directly (*e.g*., to drive a compressor) or be converted to electrical power through the use of electrical power generators. In a power cycle where the working fluid is re-used, the expanded working fluid is cooled. Cooling may be accomplished in a working fluid cooling unit (*e.g*., a heat exchanger or a condenser). The cooled working fluid can then be used for repeated cycles (*i*.*e*., compression, heating, expansion, etc.). The same pump used for compression may be used for transferring the working fluid from the cooling stage.

The present application further describes a method for detecting a leak from a container comprising sampling the air in the vicinity of the container and detecting at least one additional compound of a composition described herein with means for detecting the leak, wherein a composition described herein is contained inside the container. The term "in the vicinity of" refers to within 12 inches of the outside surface of the container. Alternatively, in the vicinity may be within 6 inches, within 3 inches or within one inch of the outside surface of the container.

A container may be any known container or system or apparatus that is filled with a composition described herein. A container may include, but is not limited to, a storage container, a transport container, an aerosol can, a fire extinguishing system, a chiller apparatus, a heat pump apparatus, heat transfer container, and a power cycle apparatus (*e.g*., an organic Rankine cycle system).

Means for detecting a leak may be performed using any known sensor designed to detect leaks. In particular, means for detecting the leak includes, but is not limited to, electrochemical, corona discharge, and mass spectroscopic leak detectors.

### EXAMPLES

The invention will be described in greater detail by way of specific examples. The following examples are offered for illustrative purposes, and are not intended to limit the invention in any manner.

### Example 1. Comparative Synthesis of 1326mxz

SbCls (10.5 g) was added to a 210 mL Hastelloy C reactor, followed by HF (49 g). The reaction mixture was heated at 100°C for 1 hour and then cooled to 0 °C. Hexachlorobutadiene (30 g) was added to the reactor and the reaction mixture was heated to 100 °C. The reaction rate was indicated by pressure increase and stabilization of the pressure indicated completion of the reaction. Similar reactions were also performed using TaCls catalyst or NbCls catalyst. Results of the comparative processes are provided in Table 1. The reactions performed using TaCls and NbCls were found to stereoselectively produce (Z)-2-chloro-1,1,1,4,4,4-hexafluoro-2-butene in a ratio of greater than about 99 : 1 (Z)-2-chloro-1,1,1,4,4,4-hexafluoro-2-butene : (E)-2-chloro-1,1,1,4,4,4-hexafluoro-2-butene.

**Table 1.**

| **Reaction #** | **1^{∗}** | **2^{∗}** | **3^{∗}** | **4^{∗}** | **5** | **6** |
|---|---|---|---|---|---|---|
| **Catalyst** | SbCl₅ | SbCl₅ | SbCl₅ | TaCl₅ | NbCl₅ | NbCl₅ |
| **Catalyst Conc. (mole percent)** | 1.5 | 2 | 2.5 | 1.5 | 1.5 | 2 |
| **Temperature (°C)** | 100 | 100 | 100 | 115 | 130 | 130 |
| **Reaction Completion Time (h)** | 18 | 10 | N/A | 4 | 9 | 6 |

| | | | | | | |
|---|---|---|---|---|---|---|
| ^{∗} Reference examples | | | | | | |

## Claims

1. A process of preparing 2-chloro-1,1,1,4,4,4-hexafluoro-2-butene, comprising reacting hexachlorobutadiene with hydrofluoric acid in the presence of a transition metal catalyst, wherein the transition metal catalyst is a niobium catalyst selected from niobium (V) chloride, niobium (IV) chloride or a mixture thereof.

2. The process of claim 1, wherein the transition metal catalyst is niobium (V) chloride.

3. The process of any one of claims 1 or 2, wherein the process is performed at a temperature of from 110°C to 135°C.

4. The process of any one of claims 1 or 2, wherein the process comprises:
i) adding the transition metal catalyst to the hydrofluoric acid to form a first mixture; and
ii) adding the hexachlorobutadiene to the first mixture to form a second mixture.

5. The process of claim 4, wherein the first mixture is heated to a temperature of from 110°C to 140°C.

6. The process of claim 5, further comprising cooling the first mixture to a temperature of from -10°C to 10°C prior to performing step ii).

7. The process of claim 6, further comprising heating the second mixture to a temperature of from 110°C to 140°C.

8. The process of claim 1 comprising:
i) adding niobium (V) chloride to hydrofluoric acid to form a first mixture;
ii) heating the first mixture to a temperature of from 125°C to 135°C;
iii) cooling the first mixture to a temperature of from -10°C to 10°C;
iv) adding hexachlorobutadiene to the first mixture to form a second mixture; and
v) heating the second mixture to a temperature of from 125°C to 135°C.

9. The process of any one of claims 1 to 8, wherein a molar excess of hydrofluoric acid is used based on 1 molar equivalent of hexachlorobutadiene.

10. The process of any one of claims 1 to 9, wherein the process of preparing 2-chloro-1,1,1,4,4,4-hexafluoro-2-butene is performed in the absence of an additional solvent component.

11. The process of any one of claims 1 to 10, wherein greater than 99 mole percent of the 2-chloro-1,1,1,4,4,4-hexafluoro-2-butene produced is (Z)-2-chloro-1,1,1,4,4,4-hexafluoro-2-butene.

12. The process of any one of claims 1 to 11, further comprising reacting the 2-chloro-1,1,1,4,4,4-hexafluoro-2-butene with a base in the presence of an alkali metal halide and a quaternary (C₄₋₁₂ alkyl)ammonium salt to form perfluorobut-2-yne.

13. The process of claim 12, wherein the base is selected from the group consisting of lithium hydroxide, sodium hydroxide, potassium hydroxide, calcium hydroxide, magnesium oxide, calcium oxide, sodium carbonate, potassium carbonate, sodium phosphate, potassium phosphate, and mixtures thereof
and/or
wherein the alkali metal halide is sodium chloride
and/or
wherein the quaternary (C₄₋₁₂ alkyl)ammonium salt is selected from the group consisting of tetrabutylammonium chloride, tetrabutylammonium bromide, tetrabutylammonium hydrogen sulfate, tetraoctylammonium chloride, tetraoctylammonium bromide, tetraoctylammonium hydrogen sulfate, trioctylmethylammonium chloride, trioctylmethylammonium bromide, tetradecylammonium chloride, tetradecylammonium bromide, and tetradodecylammonium chloride.

14. The process of claim 12 or 13, further comprising reacting the perfluorobut-2-yne with hydrogen in the presence of a hydrogenation catalyst to form 1,1,1,4,4,4-hexafluoro-2-butene, wherein the hydrogenation catalyst preferably is Lindlar's catalyst.

15. The process of claim 14, wherein the process of preparing 1,1,1,4,4,4-hexafluoro-2-butene is performed in the absence of an additional solvent component.

## Patentansprüche

1. Verfahren zur Herstellung von 2-Chlor-1,1,1,4,4,4-hexafluor-2-buten, umfassend Reagieren von Hexachlorbutadien mit Fluorwasserstoffsäure in Gegenwart eines Übergangsmetallkatalysators, wobei der Übergangsmetallkatalysator ein Niobkatalysator ist ausgewählt unter Niob(V)chlorid, Niob(IV)chlorid oder einer Mischung davon.

2. Verfahren nach Anspruch 1, wobei der Übergangsmetallkatalysator Niob(V)chlorid ist.

3. Verfahren nach einem der Ansprüche 1 oder 2, wobei das Verfahren bei einer Temperatur von 110 °C bis 135 °C ausgeführt wird.

4. Verfahren nach einem der Ansprüche 1 oder 2, wobei das Verfahren Folgendes umfasst:
i) Zusetzen des Übergangsmetallkatalysators zu der Fluorwasserstoffsäure, um eine erste Mischung zu bilden; und
ii) Zusetzen des Hexachlorbutadiens zu der ersten Mischung, um eine zweite Mischung zu bilden.

5. Verfahren nach Anspruch 4, wobei die erste Mischung auf eine Temperatur von 110 °C bis 140 °C erhitzt wird.

6. Verfahren nach Anspruch 5, ferner das Kühlen der ersten Mischung auf eine Temperatur von -10 °C bis 10 °C vor Ausführen des Schritts ii) umfassend.

7. Verfahren nach Anspruch 6, ferner das Erhitzen der zweiten Mischung auf eine Temperatur von 110 °C bis 140 °C umfassend.

8. Verfahren nach Anspruch 1, umfassend:
i) Zusetzen von Niob(V)chlorid zu Fluorwasserstoffsäure, um eine erste Mischung zu bilden;
ii) Erhitzen der ersten Mischung auf eine Temperatur von 125 °C bis 135 °C;
iii) Kühlen der ersten Mischung auf eine Temperatur von -10 °C bis 10 °C;
iv) Zusetzen von Hexachlorbutadien zu der ersten Mischung, um eine zweite Mischung zu bilden; und
v) Erhitzen der zweiten Mischung auf eine Temperatur von 125 °C bis 135 °C.

9. Verfahren nach einem der Ansprüche 1 bis 8, wobei ein molarer Überschuss von Fluorwasserstoffsäure auf der Basis von 1 Moläquivalent Hexachlorbutadien verwendet wird.

10. Verfahren nach einem der Ansprüche 1 bis 9, wobei das Verfahren zur Herstellung von 2-Chlor-1,1,1,4,4,4-hexafluor-2-buten in Abwesenheit einer zusätzlichen Lösungsmittelkomponente ausgeführt wird.

11. Verfahren nach einem der Ansprüche 1 bis 10, wobei mehr als 99 Molprozent des hergestellten 2-Chlor-1,1,1,4,4,4-hexafluor-2-butens (Z)-2-Chlor-1,1,1,4,4,4-hexafluor-2-butens sind.

12. Verfahren nach einem der Ansprüche 1 bis 11, ferner das Reagieren des 2-Chlor-1,1,1,4,4,4-hexafluor-2-butens mit einer Base in Gegenwart eines Alkalimetallhalogenids und eines quartären (C₄₋₁₂-Alkyl)ammoniumsalzes, um Perfluorbut-2-yn zu bilden, umfassend.

13. Verfahren nach Anspruch 12, wobei die Base aus der Gruppe ausgewählt wird bestehend aus Lithiumhydroxid, Natriumhydroxid, Kaliumhydroxid, Calciumhydroxid, Magnesiumoxid, Calciumoxid, Natriumcarbonat, Kaliumcarbonat, Natriumphosphat, Kaliumphosphat und Mischungen davon und/oder
wobei das Alkalimetallhalogenid Natriumchlorid ist
und/oder
wobei das quartäre (C₄₋₁₂-Alkyl)ammoniumsalz aus der Gruppe ausgewählt wird bestehend aus Tetrabutylammoniumchlorid, Tetrabutylammoniumbromid, Tetrabutylammoniumhydrogensulfat, Tetraoctylammoniumchlorid, Tetraoctylammoniumbromid, Tetraoctylammoniumhydrogensulfat, Trioctylmethylammoniumchlorid, Trioctylmethylammoniumbromid, Tetradecylammoniumchlorid, Tetradecylammoniumbromid und Tetradodecylammoniumchlorid.

14. Verfahren nach Anspruch 12 oder 13, ferner das Reagieren des Perfluorbut-2-yns mit Wasserstoff in Gegenwart eines Hydrierungskatalysators umfassend, um 1,1,1,4,4,4-Hexafluorbuten-2-buten zu bilden, wobei der Hydrierungskatalysator bevorzugt ein Lindlarkatalysator ist.

15. Verfahren nach Anspruch 14, wobei das Verfahren zur Herstellung von 1,1,1,4,4,4-Hexafluor-2-buten in Abwesenheit einer zusätzlichen Lösungsmittelkomponente ausgeführt wird.

## Revendications

1. Procédé de préparation de 2-chloro-1,1,1,4,4,4-hexafluoro-2-butène, comprenant la réaction d'hexachlorobutadiène avec de l'acide fluorhydrique en la présence d'un catalyseur métal de transition, dans lequel le catalyseur métal de transition est un catalyseur niobium sélectionné parmi le chlorure de niobium (V), le chlorure de niobium (IV) ou un mélange de ceux-ci.

2. Procédé selon la revendication 1, dans lequel le catalyseur métal de transition est le chlorure de niobium (V).

3. Procédé selon l'une quelconque des revendications 1 ou 2, dans lequel le procédé est exécuté à une température de 110 °C à 135 °C.

4. Procédé selon l'une quelconque des revendications 1 ou 2, dans lequel le procédé comprend :
i) l'addition du catalyseur métal de transition à l'acide fluorhydrique pour former un premier mélange ; et
ii) l'addition de l'hexachlorobutadiène au premier mélange pour former un second mélange.

5. Procédé selon la revendication 4, dans lequel le premier mélange est chauffé à une température de 110 °C à 140 °C.

6. Procédé selon la revendication 5, comprenant en outre le refroidissement du premier mélange à une température de -10 °C à 10 °C avant d'exécuter l'étape ii).

7. Procédé selon la revendication 6, comprenant en outre le chauffage du second mélange à une température de 110 °C à 140 °C.

8. Procédé selon la revendication 1 comprenant :
i) l'addition de chlorure de niobium (V) à de l'acide fluorhydrique pour former un premier mélange ;
ii) le chauffage du premier mélange à une température de 125 °C à 135 °C ;
iii) le refroidissement du premier mélange à une température de -10 °C à 10 °C ;
iv) l'addition d'hexachlorobutadiène au premier mélange pour former un second mélange ; et
v) le chauffage du second mélange à une température de 125 °C à 135 °C.

9. Procédé selon l'une quelconque des revendications 1 à 8, dans lequel un excès molaire d'acide fluorhydrique est utilisé sur la base d'un équivalent 1 molaire d'hexachlorobutadiène.

10. Procédé selon l'une quelconque des revendications 1 à 9, dans lequel le procédé de préparation de 2-chloro-1,1,1,4,4,4-hexafluoro-2-butène est exécuté en l'absence d'un composé solvant additionnel.

11. Procédé selon l'une quelconque des revendications 1 à 10, dans lequel plus de 99 pourcent en mole du 2-chloro-1,1,1,4,4,4-hexafluoro-2-butène produit est du (Z)-2-chloro-1,1,1,4,4,4-hexafluoro-2-butène.

12. Procédé selon l'une quelconque des revendications 1 à 11, comprenant en outre la réaction du 2-chloro-1,1,1,4,4,4-hexafluoro-2-butène avec une base en la présence d'un halogénure de métal alcalin et d'un sel (d'alkyle en C₄₋₁₂) d'ammonium quaternaire pour former du perfluorobut-2-yne.

13. Procédé selon la revendication 12, dans lequel la base est sélectionnée dans le groupe constitué de l'hydroxyde de lithium, hydroxyde de sodium, hydroxyde de potassium, hydroxyde de calcium, oxyde de magnésium, oxyde de calcium, du carbonate de sodium, carbonate de potassium, phosphate de sodium, phosphate de potassium, et des mélanges de ceux-ci et/ou
dans lequel l'halogénure de métal alcalin est le chlorure de sodium
et/ou
dans lequel le sel (d'alkyle en C₄₋₁₂) d'ammonium quaternaire est sélectionné dans le groupe constitué du chlorure de tétrabutylammonium, bromure de tétrabutylammonium, hydrogénosulfate de tétrabutylammonium, chlorure de tétraoctylammonium, bromure de tétraoctylammonium, hydrogénosulfate de tétraoctylammonium, chlorure de trioctylméthylammonium, bromure de trioctylméthylammonium, chlorure de tétradécylammonium, bromure de tétradécylammonium, et chlorure de tétradodécylammonium.

14. Procédé selon la revendication 12 ou 13, comprenant en outre la réaction du perfluorobut-2-yne avec de l'hydrogène en la présence d'un catalyseur d'hydrogénation pour former du 1,1,1,4,4,4-hexafluoro-2-butène, dans lequel le catalyseur d'hydrogénation est préférablement un catalyseur de Lindlar.

15. Procédé selon la revendication 14, dans lequel le procédé de préparation de 1,1,1,4,4,4-hexafluoro-2-butène est exécuté en l'absence d'un composé solvant additionnel.
